# FASCICULE DE BREVET EUROPEEN

(11) **EP 1 451 222 B1**
(45) Date de publication et mention de la délivrance du brevet: **10.09.2008**
(21) Numéro de dépôt: 02804246.3
(22) Date de dépôt: 04.12.2002
(51) Int. Cl.: C07K 14/54, C12N 15/24, C12N 15/63, A61K 38/20, A01K 67/027, A61P 35/00, C07H 21/00

(54) **PROTEINES A ACTIVITE INHIBITRICE DE L'IL-6**
PROTEINE MIT IL-6 INHIBITORISCHER WIRKUNG
PROTEINS WITH IL-6 INHIBITING ACTIVITY

(30) Priorité: 04.12.2001 FR 0115623
(43) Date de publication de la demande: 01.09.2004
(73) Titulaire: UNIVERSITE CLAUDE BERNARD - LYON 1, 69622 Villeurbanne Cédex (FR); Centre Leon Berard, 69373 Lyon Cedex 08 (FR); INSTITUT NATIONAL DE LA SANTE ET DE LA RECHERCHE MEDICALE (INSERM), 75654 Paris Cédex 13 (FR); Hospices Civils de Lyon, 69229 Lyon Cedex 02 (FR)
(72) Inventeur: BLAY, Jean-Yves, F-38290 Frontonas (FR); ALBERTI, Laurent, F-69210 L'Arbresle (FR)
(74) Mandataire: Sarlin, Laure V.
(86) Numéro de dépôt international: PCT/FR2002/004171
(87) Numéro de publication internationale: WO 2003/048205

(56) Documents cités:
- WO-A-94/19004
- WO-A-95/13393
- WO-A-97/10338
- WO-A1-98/15283
- WO-A2-95/00852
- US-A- 5 723 120
- WLASCHEK MEINHARD ET AL: "UVA-induced autocrine stimulation of fibroblast-derived collagenase by IL-6: A possible mechanism in dermal photodamage?" JOURNAL OF INVESTIGATIVE DERMATOLOGY, vol. 101, no. 2, 1993, pages 164-168, XP008019164 ISSN: 0022-202X
- ARCONE R ET AL: "INTERNAL DELETIONS OF AMINO ACIDS 29-42 OF HUMAN INTERLEUKIN-6 IL-6 DIFFERENTIALLY AFFECT BIOACTIVITY AND FOLDING" FEBS, vol. 288, no. 1-2, 1991, pages 197-200, XP002246561 ISSN: 0014-5793
- BRAKENHOFF J P J ET AL: "Analysis of Human IL-6 Mutants Expressed in Escherichia coli. " JOURNAL OF IMMUNOLOGY, vol. 143, no. 4, 15 août 1989 (1989-08-15), pages 1175-1182, XP001040401 ISSN: 0022-1767
- SAVINO R. ET AL EMBO J. vol. 13, no. 24, 1994, pages 5863 - 5870, XP000606356

## Description

La présente invention a pour objet des nouvelles protéines à activité inhibitrice de l'interleukine 6 (IL-6), les outils de génie génétique pour les produire, à savoir un ADN recombinant, un vecteur d'expression portant cet ADN recombinant, les micro-organismes procaryotes et les cellules eucaryotes contenant cet ADN recombinant, ainsi qu'un médicament, utile notamment comme agent anti-tumoral, contenant une de ces protéines à titre de principe actif.

L'IL-6 appartient à la famille des cytokines. Cette glycoprotéine (**SEQ ID N° 1**) possède un peptide signal de 28 acides aminés qui est clivé à la sécrétion pour donner une protéine de 184 acides aminés (E. Sporeno et al., Blood, 1996, 11, 45 10-4519) est produite par une très large gamme de cellules normales ou transformées, par exemple, les lymphocytes T ou B, les monocytes, les macrophages, les fibroblastes...

L'IL-6 est codée par un gène de 5 Kb constitué de 5 exons (**SEQ ID N° 2**) et localisé sur le chromosome 7 humain.

Des protéines antagonistes de l'interleukine 6 ont été décrites notamment par Savino et al. (EMBO J. 13, 1994, p.5863-5870) ainsi que dans les publications US5,723,120 et WO95/00852.

Brakenhoff et al. présentent des travaux sur des délétions fractionnées en N-terminal de l'IL-6 dans leur article paru dans The Journal of Immunology, 1989, 143(4), 1175-1182. Différents clones ou mutants présentant des délétions courtes, d'au maximum 35 acides aminés en *N-*terminal, sont préparés en système procaryote et en conditions dénaturantes. D'après leur résultat, les auteurs concluent que les acides aminés 31 à 34 forment une région importante pour l'activité biologique de l'IL-6 et qu'ils interviennent dans la conformation de l'IL-6 permettant la fixation de celle-ci sur un de ses récepteurs. Les auteurs ont recherché une activité antagoniste de l'IL-6 pour les mutants préparés sur la prolifération des cellules B9 et indiquent qu'ils ne l'ont pas mise en évidence (page 1180 colonne 2 ligne 43).

L'épissage de l'ARNm de l'IL-6 a été étudié notamment par Kestler et al. Dans leur article, Blood 1995, 86(12), 4559-4567, l'épissage du deuxième exon de ARNm de l'IL-6 entraîne la déletion de l'adénine située au début du troisième exon de l'ARNm de l'IL-6. De plus, aucune activité inhibitrice de l'IL-6 n'est rapportée pour la protéine issue de la traduction de cet ARNm épissée.

Or, il est intéressant de trouver des moyens pour moduler et en particulier, inhiber l'activité de l'IL-6, impliquée dans la prolifération des cellules tumorales notamment.

De façon surprenante, les inventeurs ont démontré que la protéine de séquence **SEQ ID N° 4** correspondant à la traduction de ARNm de l'IL-6 initiée à partir du deuxième ATG de l'exon 3 possède une activité inhibitrice de l'IL-6.

L'invention a donc pour objet cette protéine de séquence **SEQ ID N°** 4.

La présente invention a également pour objet les protéines correspondant à la **SEQ ID N°4** avec une queue polyhistidine en C ou N-terminal et qui se comportent également comme des inhibiteurs de l'IL-6, nommées respectivement :
- PAC3 qui présente la séquence **SEQ ID N° 3** et qui correspond à la **SEQ ID N° 4** à laquelle une tyrosine suivie d'une queue polyhistidine a été ajoutée en *C-*terminal, et
- PAC3Bis qui présente la séquence **SEQ ID N° 19** et qui correspond à la **SEQ ID N° 4** à laquelle une méthionine et une lysine suivies d'une queue polyhistidine et d'une glutamine ont été ajoutées en *N*-terminal.

Les inventeurs ont mis en évidence que la séquence en acides aminés **SEQ ID N° 4** permet d'obtenir une inhibition de l'activité de l'IL-6. L'invention a donc pour objet l'utilisation d'une protéine consistant en la séquence en acides aminés **SEQ ID N° 3** ou **4** ou **19** pour la fabrication d'un médicament destiné à inhiber l'activité de l'IL-6, et en particulier d'agents anti-cancéreux.

La présente invention a également pour objet tout acide nucléique qui comprend ou est constitué par un enchaînement de nucléotides codant pour les protéines selon l'invention (**SEQ ID N° 3, SEQ ID N° 4 et SEQ ID N° 19**). Il peut s'agir de séquences d'origine naturelle ou artificielle, et notamment d'ADN génomique, d'ADNc, d'ARNm, de séquence hybrides ou de séquences synthétiques ou semi-synthétiques. Cet acide nucléique peut être d'origine humaine, animale, végétale, bactérienne, virale ... Il peut être obtenu par toute technique connue de l'homme du métier, et notamment par criblage de banques, par synthèse chimique ou encore par des méthodes mixtes incluant la modification chimique ou enzymatique de séquences obtenues par criblage de banques. Avantageusement, l'acide nucléique selon l'invention est un ADNc.

Les acides nucléiques selon l'invention peuvent notamment être préparés par synthèse chimique et génie génétique en utilisant les techniques bien connues de l'homme du métier et décrites, par exemple, dans SAMBROOK et al. "Molecular Cloning : a Laboratory Manual" publié en 1989 par Cold Spring Harbor Press, NY, 2ème édition.

Par exemple, la synthèse des séquences d'ADNc selon l'invention peut être effectuée par amplification des ARNm de cellules humaines à l'aide de la méthode PCR (de l'anglais "Polymerase Chain Reaction"), comme décrit, par exemple, par GOBLET *et al.* (Nucleic Acid Research, 17, 2144, 1989) en utilisant des oligonucléotides synthétiques comme amorces, définis à partir de la séquence d'ADN **SEQ ID N°2** de l'IL-6.

Des amorces appropriées pour la synthèse de la protéine PAC3 sont, par exemple, les oligonucléotides suivants :
- amorce 5'
   **SEQ ID N°7 :** CCT TGG ATC CAT GGC TGA AAA AGA TGG
   crée un site de restriction BamHI en 5' du deuxième ATG du troisième exon du gène de l'IL-6
- amorce 3' rend silencieux le codon stop du gène de l'IL-6 puis crée une queue de six histidines avec un codon stop et un site de restriction EcoRI sur le codon stop du gène de l'IL-6

Le fragment d'acides nucléiques amplifié peut ensuite être cloné selon les techniques décrites dans AUSUBEL et al. (Current Protocols in Molecular Biology, Green Publishing Associates and Wiley Interscience, New-York, 1989, Mises à jour jusqu'en 1997, chapter 3).

Les protéines selon l'invention peuvent être obtenues par la technique du génie génétique qui comprend les étapes de :
- culture d'un micro-organisme transformé ou de cellules eucaryotes transformées à l'aide d'un vecteur d'expression portant une séquence d'acides nucléiques selon invention ; et
- récupération de la protéine produit par le dit micro-organisme ou lesdites cellules eucaryotes.

Cette technique est bien connue de l'homme du métier. Pour plus de détails la concernant, on pourra se référer à l'ouvrage ci-après : Recombinant DNA Technology I, Editors Ales Prokop, Raskesh K Bajpai ; Annals of the New-York Academy of Sciences, volume 646, 1991.

Elles peuvent également être préparées par les synthèses peptidiques classiques bien connues de l'homme du métier.

L'invention a également pour objet des micro-organismes procaryotes et les cellules eucaryotes transformés à l'aide d'un vecteur d'expression contenant une séquence d'ADN selon l'invention. Ce vecteur d'expression, qui peut être, par exemple, sous la forme d'un plasmide, cosmide ou phage, doit comporter, outre la séquence d'ADN de l'invention, les moyens nécessaires à son expression, tels que notamment un promoteur, un terminateur de transcription, une origine de réplication et de préférence un marqueur de sélection. La transformation des micro-organismes et des cellules eucaryotes est une technique bien connue de l'homme du métier qui pourra aisément déterminer, en fonction du micro-organisme à transformer, les moyens nécessaires à l'expression de la séquence d'ADN selon l'invention.

Comme micro-organisme procaryote, on peut citer *E. coli* et comme eucaryote la levure *Saccharomyces cerevisiae.*

A titre d'exemples de cellules eucaryotes qui conviennent aux fins de l'invention, on peut citer notamment les cellules de singe COS, les cellules d'ovaire de hamster chinois CHO, les cellules d'insectes Sf9, la lignée de lymphome T humain Jurkat, etc., toutes étant répertoriées à l'ATCC.

De manière avantageuse, on pourra utiliser un système mettant en oeuvre un vecteur de type baculovirus. Ce système permet de produire des protéines étrangères aux cellules (cellules Sf9) d'un insecte (*Spodoptera Frugiperda*) en utilisant une recombinaison *in vivo* entre un vecteur de transfert (plasmide) qui contient la séquence d'ADN étrangère, d'une part et d'autre part, le génome d'un virus. (O'Reilly, D., L.K. Miller, and V.A. Luckow. 1992. Baculovirus expression vectors: A Laboratory manual. W.H. Freeman and Compan, New York, Y.)

L'invention a également pour objet les micro-organismes procaryotes et les cellules eucaryotes cotransformées avec des vecteurs d'expression contenant la séquence d'ADN codant pour la protéine selon l'invention, lesdits vecteurs d'expression contenant de plus des moyens utiles à leur expression, y compris dans le système double-hybride en levure.

L'invention a également pour objet des animaux transgéniques exprimant un transgène de la protéine PAC3, PAC3Bis ou de la protéine de **SEQ ID N° 4** selon l'invention.

Ces animaux transgéniques pourront servir de modèles d'étude in vivo de la perturbation du cycle cellulaire et de la prolifération par l'absence ou la surexpression du gène de la protéine selon l'invention ou de formes tronquées ou mutées de cette protéine.

Ces animaux transgéniques sont obtenus par des techniques bien connues de l'homme du métier, telles que celles décrites dans Manipulating the mouse embryo : a laboratory manual, HOGAN B., BEDDINGTON R,COSTArifïNI F. & LACY E. Cold Spring Harbor laboratory press, second édition, 1994.

A titre d'animaux, on préfère les mammifères tels que la souris ou le rat.

La protéine de séquence **SEQ ID N°** 4, la protéine PAC3 et la protéine PAC3Bis, sont particulièrement intéressantes sur le plan thérapeutique. Leur activité thérapeutique est liée plus particulièrement à leur aptitude à inhiber l'activité de l'IL-6. Ces protéines seront en particulier utiles comme agent anti-inflammatoire pour le traitement de toute pathologie inflammatoire ou infectieuse où l'II-6 est démontrée jouer un rôle physiopathologique, pour le traitement des maladies où il est nécessaire d'inhiber la prolifération des cellules tumorales bénignes ou malignes dont la croissance est stimulée par l'IL-6, ou d'induire la mort des cellules dont la survie est dépendante de la présence d'IL-6, ou encore d'inhiber la dissémination métastatique des cellules tumorales dont la dissémination est induite par l'IL-6, de manière autocrine, paracrine ou endocrine, notamment les lymphomes, leucémies, myélomes, carcinomes, sarcomes, le sarcome de Kaposi, la maladie de Castelman, chez les patients atteints de tumeurs malignes ; où il est nécessaire d'inhiber l'effet protecteur de l'IL-6 sur la mort des cellules tumorales induite par les agents cytotoxiques (chimiques, biologiques ou physiques) ou cytostatiques ; où il est nécessaire d'inhiber les propriétés proinflammatoires, cachectisantes et régulatrices de l'hématopoïèse de l'IL-6 résultant d'une production anormale de cette protéine, notamment les syndromes inflammatoires chroniques dysimmunitaire (polyarthrite, lupus érythémateux disséminé), dans les connectivites, dans les infections graves, par exemple de type viral ou bactérien, associées à une hyperproduction d'IL-6 dans des tumeurs bénignes ou malignes d'origine hématologique, épithéliale, conjonctive, par exemple ; où il est nécessaire d'inhiber les remaniements osseux, notamment l'ostéolyse, induits par l'IL-6, dans les maladies dégénératives, inflammatoires ou tumorales, par exemple ; où il est nécessaire d'inhiber les propriétés immunologiques de l'IL-6 (immunostimulantes ou immunosuppressives) dans les situations cliniques où la dérégulation de la production d'IL-6 joue un rôle physiopathologique, notamment les syndromes dysimmunitaires, les maladies infectieuses et les affections néoplasiques de type leucémies, myélomes, carcinomes ou sarcomes.

L'invention concerne également la protéine de séquence **SEQ ID N° 4,** la protéine PAC3 et la protéine PAC3Bis pour leur utilisation en tant que médicament ainsi que les compositions pharmaceutiques contenant la protéine de séquence **SEQ ID N° 4**, la protéine **PAC3** ou la protéine PAC3Bis, en association avec au moins un excipient pharmaceutique convenable.

Ces compositions pharmaceutiques sont préparées selon les techniques classiques bien connues de l'homme de l'art. Les excipients pharmaceutiques sont choisis selon la forme pharmaceutique et le mode d'administration souhaité, par exemple, administration orale, sublinguale, sous-cutanée, intramusculaire, intra-veineuse, topique ... A titre d'exemple, la protéine ou les fragments peptidiques selon l'invention peuvent être formulés sous forme injectable, en association avec un ou plusieurs excipients pharmaceutiquement acceptables appropriés et dans un solvant physiologiquement acceptable tel que l'eau ou le sérum physiologique. Chaque dose unitaire peut contenir, par exemple, entre 10 et 500 mg de principe actif, afin d'obtenir l'effet thérapeutique ou prophylactique désiré.

La présente invention a aussi pour obj et l'utilisation de la protéine de séquences **SEQ ID N° 4**, de la protéine PAC3 ou de la protéine PAC3Bis pour la préparation de médicaments anti-tumoraux ou anti-cancéreux et en particulier destinés à prévenir ou traiter les maladies IL-6 dépendantes, et en particulier les maladies ci-dessus mentionnées, et plus particulièrement les lymphomes, leucémies, myélomes, carcinomes, sarcomes.

L'invention va maintenant être décrite en détail à l'aide de l'exposé expérimental ci-après.

Une grande partie des techniques décrites dans ces exemples, bien connues de l'homme du métier, est exposée en détail dans l'ouvrage de SAMBROOK *et al. (supra)* ou dans l'ouvrage de AUSUBEL *et al. (supra).*

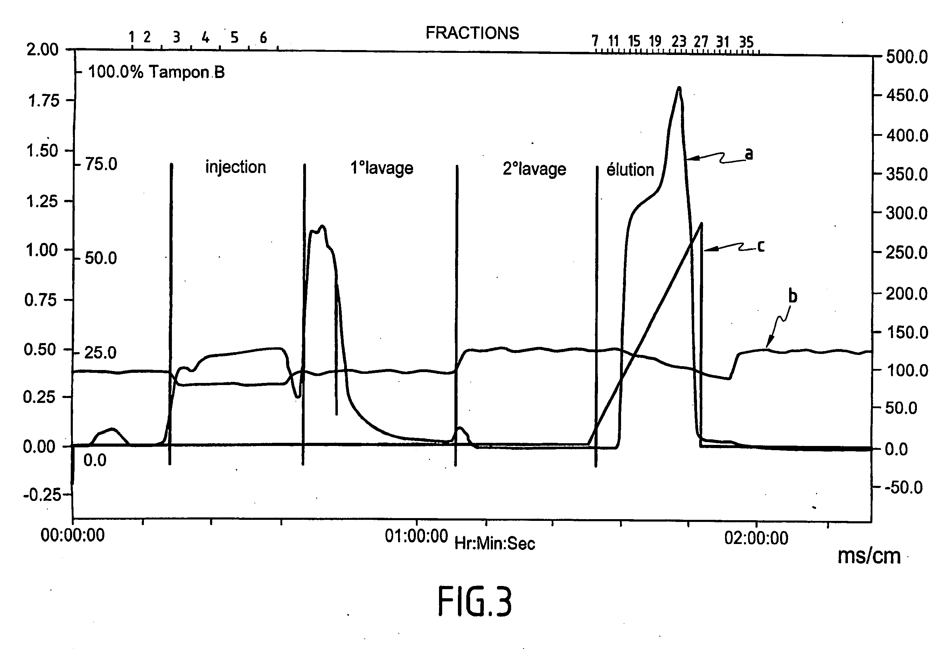

La description ci-après sera mieux comprise à l'aide des **fig. 1** à **10** sur lesquelles :
- la **fig.1** représente l'alignement des séquences obtenu à l'aide des amorces **SEQ ID n° 5** à n° 16 sur le vecteur KAE15.8.
- la **fig. 2** représente la carte du plasmide de transfert de la protéine PAC3,
- la **fig. 3** représente le profil de chromatographie utilisé lors de la purification de la protéine PAC3,
- la **fig. 4A** représente la protéine PAC3 révélée par colorimétrie argentique,
- la **fig. 4B** représente la caractérisation par Western blot de la protéine PAC3 détectée par un anticorps anti-histidine et révélée par chimiluminescence,
- les **fig. 5A****, SB, 5C** représentent les résultats obtenus avec la protéine PAC3 sur l'inhibition de la prolifération des cellules U266,
- la **fig.6** représente les résultats obtenus avec la protéine PAC3 sur la différenciation des progéniteurs CD34+,
- La **fig. 7** représente le profil de chromatographie utilisé lors de la purification de la protéine PAC3Bis.
- La **Fig. 8A** représente une autoradiographie du Western Blot de la protéine PAC3Bis.
- La **Fig. 8B** représente le même gel présenté à la **Fig. 8A** mais coloré à l'argent.
- La **Fig. 9A** représente la caractérisation par Western Blot des différentes étapes de digestion de la pertinence PAC3Bis.
- La **Fig. 9B** représente une autoradiographie du Western Blot de fractions obtenues après digestion.
- La **Fig. 9C** représente les mêmes fractions qu'à la **Fig.9B** mais coloré à l'argent.
- La **Fig. 10** représente les résultats obtenus avec la protéine PAC3Bis et la protéine de séquence **SEQ ID N°** 4 sur la prolifération des cellules U266.

On décrira ci-après plus en détails, une méthode de production et de purification de la protéine PAC3 de **SEQ ID n°3** et des protéines de **SEQ ID N°** 4 et de **SEQ ID N° 19** (PAC3Bis), ainsi que différents tests mettant en évidence leur activité biologique, notamment sur la prolifération de la lignée U266 de myélome.

### A - PRODUCTION ET PURIFICATION DE LA PROTEINE PAC3

### A.1- Extraction de l'ARN

### A.1.1 - Extraction de l'ARN

Après culture jusqu'à 80 % de confluence de la lignée tumorale CLB-TUG (déposée à la CNCM Paris, France le 13 novembre 2001 sous le numéro I-2749, cellule d'adénocarcinome de type fibroblastique établie à partir d'un ganglion lymphatique drainant une tumeur primaire d'adénocarcinome du rein d'un homme de 40 ans), le milieu de culture est éliminé, puis les cellules sont décollées par trypsination, pour former un culot sec de 10⁶ cellules. Les cellules sont alors lavées trois fois en tampon de sel de phosphate (PBS). Le culot cellulaire est ensuite resuspendu dans 1 ml de solution de TRIzol (solution commerciale de Gibco) et incubé pendant 5 minutes à température ambiante. A la fin de cette incubation, 0,2 ml de chloroforme saturé en eau sont additionnés à cette suspension. Le tout est mélangé par agitation vigoureusement pendant 15 secondes, puis l'émulsion est incubée pendant 5 minutes à température ambiante. Les différentes phases sont séparées par une centrifugation de 15 minutes à 12000 g et à 4 °C. La phase aqueuse comprenant l'ARN est transférée dans un nouveau tube afin de précipiter l'ARN à l'aide de 0,5 ml d'isopropanol. Le mélange est incubé pendant 1 heure à - 20 °C, puis centrifugé pendant 15 minutes à 12 000 g à 4 °C. Le culot d'ARN est lavé par 1 ml d'éthanol à 75 % puis centrifugé pendant 10 minutes à 12000 g et à 4 °C. Après élimination de l'éthanol, le culot est solubilisé à une concentration de 1 mg.ml⁻¹ dans une solution d'eau à 0,1 % de diéthylpyrocarbonate (DEPC).

### A.1.2 - Préparation de l'acide désoxyribonucléique complémentaire (ADNc)

Pour la synthèse de l'acide désoxyribonucléique complémentaire (ADNc), le système " Expand Reverse Transcriptase " de Boerhinger Manheim (Boerhinger Manheim, Meylan, France) est utilisé selon les recommandations du fournisseur.
20 µl d'une solution d'eau à 0,1 % de DEPC contenant 1 µg d'ARN, 50 pM d'oligonucléotide (dT)18 (Genset, Paris, France) sont dénaturés à 65 °C pendant 10 minutes. Parallèlement, est préparée, dans un volume final de 20 µl, une solution comprenant du tampon de l'Expand reverse 1X, 10 mM de DTT, 1 mM de chacun des quatre désoxyribonucléotides (dATP, dCTP, dGTP, dTTP), 20 UI d'inhibiteur de Rnase et 50 UI d'Expand Reverse. Les deux solutions sont mélangées puis incubées pendant 60 minutes à 42 °C.

### A.2 - PCR (Polymerase Chain Reaction)

### A.2.1 - Amorces de PCR

Les couples d'amorces utilisées au cours des PCR sont les suivantes :

### Premier couple d'amorces :

### - amorce 5'

**SEQ ID N° 5** : GAG AAG CTT TAG CGG CCG CCC AGG AGC CCA GCT crée un site de restriction HinD III en 5' du premier ATG du gène de l'IL-6.

### - amorce 3'

**SEQ ID N° 6** : GAA TGC CCG GGA AAC TCG AGA ATC TGA GGT GCC C crée un site de restriction Sma I en 3' du codon stop du gène de l'IL-6.

### Deuxième couple d'amorces :

### - amorce 5'

**SEQ ID N° 7** : CCT TGG ATC CAT GGC TGA AAA AGA TGG
crée un site de restriction BamHI en 5' du deuxième ATG du troisième exon du gène de l'IL-6.

### - amorce 3'

rend silencieux le codon stop du gène de l'IL-6 puis crée une queue de six Histidines avec un codon stop et un site de restriction EcoR I sur le codon stop du gène de l'IL-6.

### Amorces de séquençage:

**SEQ ID N° 9** : AGC TAT GAA CTC CTT CTC C amorce de séquence à partir de la base 30 dans le sens de lecture du gène de l'IL-6.

**SEQ ID N° 10 :** GTT CTG AAG AGG TGA GTG GC
amorce de séquence à partir de la base 187 sur le brin complémentaire du gène de l'IL-6 épissé.

**SEQ ID N° 11**: AGG TAT ACC TAG AGT ACC TCC
amorce de séquence à partir de la base 401 dans le sens de lecture du gène de l'IL-6.

**SEQ ID N° 12** : AAC TCG AGA ATC TGA GGT GC
amorce de séquence à partir de la base 697 sur le brin complémentaire du gène de l'IL-6.

**SEQ ID N° 13** : TCG AGG TCG ACG GTA TC
amorce de séquence s'hybride en 5' du site de clonages multiples du plasmide KS+.

**SEQ ID N° 14** : GCG AGA TCT TGA TCA CCT AG
amorce de séquence s'hybride en 3' du site de clonages multiples du plasmide KS+.

**SEQ ID N°15** : GTA ATA CGA CTC ACT ATA GGG C
amorce de séquence s'hybride en 5' du site de clonages multiples du plasmide KS+.

**SEQ ID N° 16** : GCT TGT TCC TCA CTA CTC TC
amorce de séquence à partir de la base 258 sur le brin complémentaire du gène de l'IL,-6.

### A.2.2 - Amplification des ADNc par PCR

1 µg de solution d'ADNc à tester est ajouté à 25 µl d'une solution contenant 0,2 mM de dNTP (Promega, Lyon, France) et 300nM de chacune des deux amorces (**SEQ ID N° 5 et N° 6**) choisies. Extemporanément à la réaction PCR, 25 µl d'une solution comprenant 100 mM de KCI, 20 mM de Tris-HCl (pH = 7,5), 0,5 % de Tween 20, 1,5 mM de MgCl₂, 2,6 U d'Expand Polymerase (Boehringer) sont alors additionnés aux 25 premiers µl. Les cycles de PCR et les températures d'hybridation varient en fonction des amorces utilisées comme présenté au **TABLEAU 1.**

**TABLEAU 1**

| Amorces | Concentration | Cycles |
|---|---|---|
| SEQ ID N° 5 | 7,5 nm /ml | 30 minutes à 94 °C |
| | | 30 minutes à 58 °C |
| SEQ ID N° 6 | | 45 minutes à 72 °C |
| | | 35 cycles |
| SEQ ID N° 7 | 7,5 nm /ml | 30 minutes à 94 °C |
| | | 30 minutes à 58 °C |
| SEQ ID N° 8 | | 45 minutes à 72 °C |
| | | 35 cycles |

Chaque PCR débute par une dénaturation de 2 minutes à 94 °C et finit par une élongation de 7 minutes à 72 °C.
Du quinzième' au trente-cinquième cycle, le temps de l'élongation est augmenté de 20 secondes par cycle.
La PCR est faite dans un appareil thermocycle Eppendorf gradient (Merck, Strasbourg, France). Les produits de réaction de PCR sont analysés sur un gel 1 % d'agarose en TBE (Tris bases 45mM, EDTA 10 mM).

### A.2.3 - Purification des fragments PCR

Les produits de PCR sont purifiés par Wizard de Promega, à partir d'un gel d'agarose 0, 1 % en tampon TAE (Tris-HCl 40 mM, EDTA 1 mM). Après migration, les bandes intéressantes sans matériel contaminant sont prélevées à l'aide d'un scalpel stérile. Le gel contenant l'ADN est découpé sous U.V. Le morceau de gel est incubé pendant 5 minutes à 65° C dans 1 ml de solution de résine. Celle-ci est récoltée sur filtre, puis lavée par 2 ml d'isopropanol à 80 %. Après centrifugation (30 secondes à 12000 g), l'ADN purifié est hydraté par 40 µl d'eau, puis élué par centrifugation (30 secondes à 12 000 g).

### A.2.4 - Clonage du produit PCR

Après purification, les bandes PCR doivent être clonées dans un plasmide, afin de les amplifier, permettant ainsi de les séquencer et de les sous-cloner dans différents plasmides d'expression. A l'aide de la PCR, deux sites de restriction (HinD III en 5' et Sma 1 en 3') permettant de cloner le fragment dans le site de clonage multiple du plasmide "Blue script" SK⁺ (Startagene, Ozyme, Montigny, France), ont été créés. 1 µg d'ADN est mis à digérer pendant 4 heures à 25 °C dans une solution comprenant 50mM de K(C₂H₃O₂), 20 mM de Tris (pH = 7,9), 10 mM de Mg(C₂H₃O₂)₂, 1mM de DTT et 80 U d'enzyme Sma I (Biolabs, Ozyme, Saint Quentin en Yvelines, France). 10 U d'enzyme HinD III sont alors ajoutés, et la solution est incubée pendant 12 heures à 37 °C. Parallèlement 0,5 µg de plasmide SK⁺ sont digérés de la même façon.

Les produits de digestion sont purifiés avec le kit Compass (American Bioanalytical, Natick, USA). L'ADN est dilué dans 3 volumes d'une solution saline (fournie avec le kit) additionnée de 5 µl de matrice de silane. Après 5 minutes d'incubation à température ambiante, la solution est centrifugée (30 s. à 12000g). La résine culottée est lavée par 1 ml de solution de lavage (fournie avec le kit) qui est ensuite éliminée par deux centrifugations successives de 30 secondes à 12000 g. Après 5 minutes à température ambiante, la résine est resuspendue dans 12 µl d'eau. Après incubation de 5 minutes à 65 °C, la suspension est centrifugée pendant 1 minute à 12 000 g, afin de prélever le surnageant comprenant l'ADN.

Le fragment digéré et purifié et le plasmide sont incubés dans une solution comprenant 50 mM de Tris (pH = 7,8), 10 mM de MgCl₂, 10 mM de DTT, 1 mM d'ATP, 50 mg.ml⁻¹ de BSA et 24000 U de T4 ligase (Biolabs). La solution est incubée pendant 16 heures à 4 °C.

La solution de ligation est ensuite transformée dans une bactérie compétente JM109 (Promega), afin d'obtenir une quantité de plasmide suffisante permettant le séquençage du produit PCR inséré dans le plasmide.

### A.2.5 - Séquençage et analyse des séquences

Le séquençage de l'insert est réalisé à l'aide d'un séquenceur automatique 373A Applied Biosystem (Roissy, FRANCE). Le principe du séquençage automatique repose sur la détermination par un laser d'une suite de bases fluorescentes incorporées dans des brins d'ADN néosynthétisés à partir de la séquence de référence par PCR.

Par cette PCR, on synthétise à partir d'amorces choisies dans le **TABLEAU 2** ci-après en fonction de la séquence à analyser des brins d'ADN dans lesquels des bases fluorescentes sont incorporées lors de la synthèse du brin complémentaire.

**TABLEAU 2**

| Amorces | Concentration | Cycles |
|---|---|---|
| SEQ ID N° 9 | 10 µM | 30 secondes à 96 °C |
| | | 30 secondes à 55 °C |
| | | 4 secondes à 68 °C |
| | | 35 cycles |
| SEQ ID N°10 | 10 µM | 30 secondes à 96 °C |
| | | 30 secondes à 55 °C |
| | | 4 secondes à 68 °C |
| | | 35 cycles |
| SEQ ID N° 11 | 10 µM | 30 secondes à 96 °C |
| | | 30 secondes à 55 °C |
| | | 4 secondes à 68 °C |
| | | 35 cycles |
| SEQ ID N° 12 | 10 µM | 30 secondes à 96 °C |
| | | 30 secondes à 55 °C |
| | | 4 secondes à 68 °C |
| | | 35 cycles |
| SEQIDN°13 | 10 µM | 30 secondes à 96 °C |
| | | 30 secondes à 55 °C |
| | | 4 secondes à 68 °C |
| | | 35 cycles |
| SEQ ID N° 14 | 10 µM | 30 secondes à 96 °C |
| | | 30 secondes à 55 °C |
| | | 4 secondes à 68 °C |
| | | 35 cycles |
| SEQ ID N° 15 | 10 µM | 30 secondes à 96 °C |
| | | 30 secondes à 55 °C |
| | | 4 secondes à 68 °C |
| | | 35 cycles |
| SEQ ID N° 16 | 10 µM | 30 secondes à 96 °C |
| | | 30 secondes à 55 °C |
| | | 4 secondes à 68 °C |
| | | 35 cycles |

1 µg de plasmide à tester préparé par midi-prép est ajouté à 20µl d'une solution contenant du tampon Fs (Applied Biosystem, St Quentin en Yvelines, France) et 0,5 µM d'une amorce de séquence (TABLEAU 2).

Chaque PCR débute par une dénaturation de 3 min. à 96 °C et finit par une élongation finale de 4 min. à 68 °C.

A l'aide du logiciel Vector NTI de la société Informax (Oxford, U.K.), nous alignons l'ensemble des séquences obtenues par les différentes amorces à l'aide du logiciel Vector NTI de la société Informax (Oxford U.K.), l'alignement de l'ensemble des séquences obtenues par les différentes amorces sur le plasmide KAE15.8 est effectué.

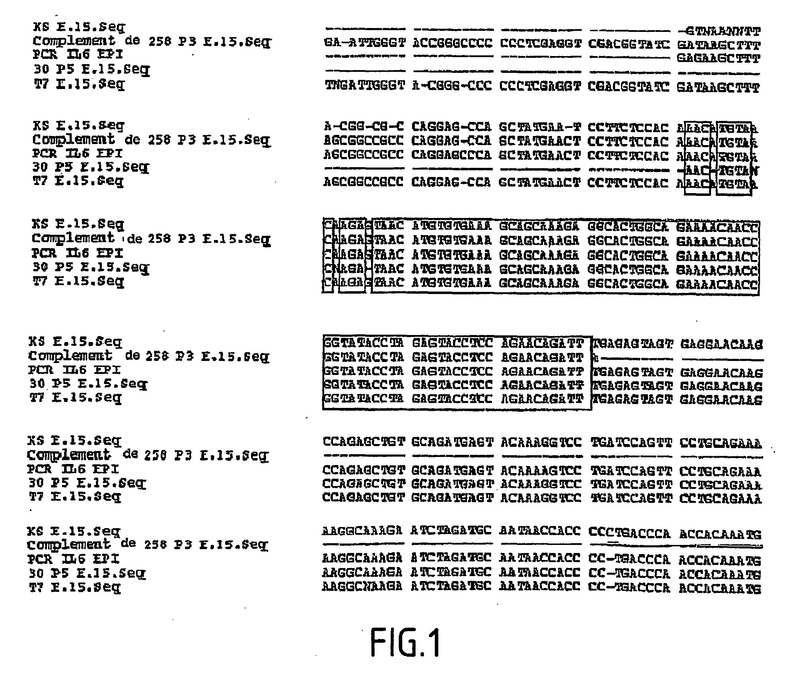

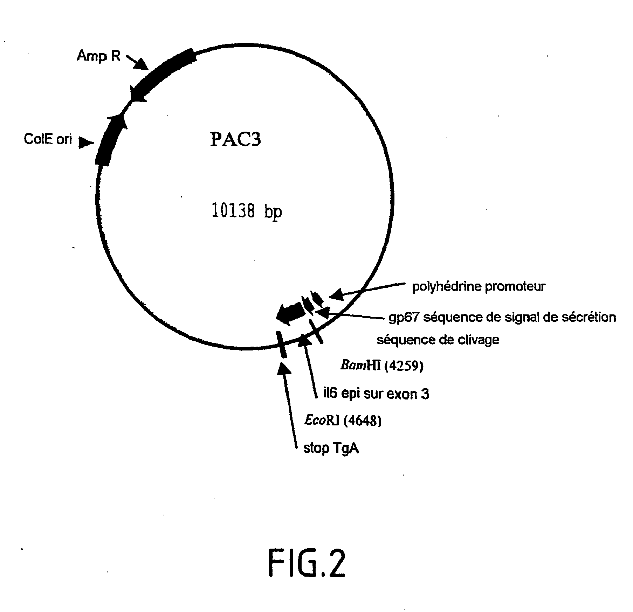

Cet alignement permet de comparer les séquences entre elles et de les comparer par rapport à la séquence théorique attendue qui est ici nommée PCR IL-6 Epi. Le fait d'utiliser plusieurs amorces pour séquencer le même insert, nous permet de nous affranchir des erreurs de lecture du séquenceur automatique. On pourra se référencer aux **fig. 1** et **2****.**

Ce plasmide KAE15.8 va servir pour toutes les autres constructions plasmidiques.

### A.3 - Préparation des baculovirus

Le système de production de virus utilisé est le Baculogold^{®} transfection Kit (PharMingen, Becton Dickinson, Paris , France). La préparation de baculovirus est réalisée en deux temps : dans un premier temps, le vecteur de transfert est construit, puis dans un second temps, des virus sont obtenus par recombinaison homologue entre le vecteur de transfert et un baculovirus linéaire dans des cellules sf9.

### A.3.1 - Préparation du vecteur de transfert

Sur le plasmide KAE15.8 précédemment obtenu et séquencé, une PCR est réalisée à l'aide du couple d'amorces **SEQ ID N° 5** et **SEQ ID N**° **6**. La PCR, l'analyse des fragments et leur purification sont réalisées comme précédemment décrit.

La digestion des fragments PCR purifiés et du plasmide de transfert pAcGP67-B est réalisée par les enzymes BamHI et EcoRI (biolab) pendant 4 heures à 37 °C. La ligation de l'insert est identique à celle précédemment décrite. Les constructions plasmidiques sont ensuite transformées dans JM109 puis séquencées.

Les vecteurs de transfert dont la séquence est correcte, sont amplifiés afin d'obtenir 1 mg de plasmide dépourvu d'endonucléase à l'aide du kit EndoFree^{®} plasmid Mega Kits de Qiagen. (Courtaboeuf, France)

Ce lot de plasmide va servir pour la co-transfection du vecteur de transfert et du baculovirus linéaire dans les cellules Sf9. La carte de ce vecteur de transfert est présentée à la **fig. 2**.

### A.3.2 - Préparation et sélection des virus

Les baculovirus sont obtenus par co-transfection d'un vecteur de transfert et d'un baculovirus linéaire dans une cellule Sf9. Après recombinaison homologue, la cellule produit des virions réplicatifs produisant la protéine d'intérêt.

### A.3.2.1- Co-transfection

2.10⁶ cellules Sf9 sont co-transfectées par 0,5 µg de baculogold et 5 µg de vecteur de transfert par du sulfate de calcium. Après 4 heures d'incubation à 27 °C, le milieu de transfection est remplacé par du milieu de culture SF900 Il (Gibco) complémenté par 5% de sérum de veau nouveau né (Hyclone, Perbio, Bezon, France). Au bout de 5 jours d'incubation à 27 °C, le surnageant de culture comprenant les virus est prélevé, afin de cloner ceux-ci par la technique de plaque assay.

### A.3.2.2 - Clonage des particules virales par plaque assay

Les particules virales sont clonées par sélection de plage de lyse en milieu gélifié. 7.10⁶ cellules Sf9 sont ensemencées en boite de culture de diamètre 100 mm. Une boite de culture est préparée par dilution du surnageant de culture contenant les particules virales. Une gamme de dilutions est réalisée de 10⁻¹ à 10⁻⁶ suivant le titre viral de surnageant de culture. Les cellules sont incubées pendant 1 heure à 27 °C avec la dilution, puis une solution à 2 % d'agarplaque (préparé en milieu de culture sans protéine) est coulée sur chaque boite de culture. Au bout de 10 minutes à température ambiante, l'ensemble de la boite se gélifie emprisonnant les cellules dans un milieu de culture à 1 % d'agarplaque. L'ensemble est mis à incuber pendant 12 jours à 27 °C.

Le virus étant bloqué par l'agarplaque, celui-ci ne se développe plus que par contamination de cellule à cellule formant ainsi des plages de lyse cellulaire pouvant être lues à l'oeil nu. Si la dilution virale est suffisante, une plage de lyse correspond à un virus. Les plages de lyse sont prélevées à l'aide d'un cône de pipette, puis transférées dans 1 ml de milieu de culture, afin d'éluer les particules virales de l'agarose par une incubation de 12 heures à 4 °C sous agitation.

300 µl de cet éluat va infecter 5.10⁶ cellules en boîte de culture de diamètre 100 mm en 3 jours à 27 °C. Le surnageant de culture est conservé à 4 °C et les cellules sont lavées avec du PBS, culottées puis lysées, afin de contrôler, sur gel d'acrylamide SDS-page (Gibco), la production de la protéine d'intérêt par le virus.

### A.3.3 - Caractérisation des virus par gel d'acrylamide SDS-page

2.10⁷ cellules sont lysées par 1 ml de tampon de lyse (10 mM Tris-HCl pH = 7,5; 130 mM NaCl; 1% Triton X-100 10 mM NaF; 10 mM NaPi; 10 mM NaPPi; 10 µg/ml phenanthroline; 10 µg/ml aprotinine; 10 µg/ml leupeptine; 1 mM PMSF) pendant 45 minutes à 4 °C. Le lysat est ensuite clarifié par une centrifugation de 45 minutes à 40 000 g.

30 µl de lysat sont dénaturés pendant 5 minutes à 95 °C, en présence de tampon de charge (2,5 % SDS, 5 % β-mercaptoéthanol, 1 mM Tris pH = 7,4, 1 mM EDTA, 20 % glycérol, 5 % de bleu de coomassie à 1 %). Les échantillons dénaturés font l'objet d'une électrophorèse de 2 heures à 120 V dans un gel SDS à 12 % d'acrylamide (Gibco). Après migration, les protéines sont transférées sur une membrane de PVDF (Fluorure de PolyVinyliDène de Millipor, Poly-labo, Strasbourg) par un transfert humide de 45 minutes à température ambiante à 0,350 A dans un tampon comprenant 25 mM de Tris-HCl, 192 mM de glycine, pH = 8,3. Pour les gels à 12 % d'acrylamide, 20 % de méthanol sont ajoutés à ce tampon.

Les protéines transférées sont identifiées par l'intermédiaire de leur queue poly histidine, par un anticorps monoclonal anti-histidine directement couplé à une phosphatase alcaline (Invitrogen, Gibco, France). L'ensemble est ensuite visualisé par un procédé de chimiluminescence (Tropix, Perkin Elmer).

### A.4 - Production et purification de la protéine PAC3

### A.4.1 - Production

Celle-ci est réalisée par infection de cellules Sf9 en suspension. 1 1 de milieu de culture SF900 II sérum free (Gibco) est ensemencé à l'aide de 2.10⁶ cellules.ml⁻¹ et 1 ml de suspension virale à 10⁸ pfu.ml⁻¹. L'ensemble est mis à incuber pendant 4 jours à 27 °C, sous agitation douce. A la fin de la culture les cellules sont culottées par une centrifugation à 10 000 g pendant 5 minutes et le surnageant est stocké à 4 °C.

### A.4.2 - Purification

La première étape consiste à préparer l'échantillon afin qu'il puisse être compatible avec la colonne de chromatographie. Pour cela une première dialyse est réalisée contre 20 volumes de tampon phosphate 20mM pH = 7,4 à travers une membrane de dialyse dont le point de coupure est de 15 KDa. Au bout d'une nuit de dialyse à 4 °C, sous agitation douce, le dialysat est prêt à être purifié sur colonne d'affinité HITrap chelating 5 ml de Pharmacia. Cette colonne est composée de sépharose sur laquelle est greffée une pince pouvant chélater différents types de métaux bivalents. Dans cette purification la pince est chargée avec du cuivre, conférant ainsi une affinité pour des chaînes poly-histidine.

### A.4.2.1 - Chromatographie

La colonne est montée sur le système de chromatographie HR de Biorad stabilisé à 4 °C en chambre froide. Le débit de la colonne est limité à 1 ml.min⁻¹. avec une pression maximum de 100 psi.

Dans un premier temps la colonne est lavée et activée par un premier lavage par 5 volumes d'eau distillée, puis activée par 0,5 volume d'une solution de sulfate de cuivre à 100 mM . Le cuivre non fixé est éliminé par 10 volumes d'eau distillée. La colonne est stabilisée par 10 volumes de tampon phosphate 20 mM pH = 7,4, 1 M NaCl, 10 mM d'inmidasole.

La purification se fait à l'aide de plusieurs passages successifs afin de ne pas saturer la colonne.

Chaque passage débute par une stabilisation de la ligne de base des U.V. à 280 nm à l'aide de 2 volumes de tampon phosphate 20 mM pH=7,4, 1 M NaCl, 10 mM d'imidazole, puis 4 volumes de dialysat sont injectés. Plusieurs lavages sont réalisés par 6 volumes de tampon phosphate 20 mM pH=7,4, 1 M NaCl, 10 mM d'imidazole et 6 volumes de tampon phosphate 20 mM pH=7,4, 1M NHCl₄.

Les protéines sont éluées de la colonne à l'aide de 4 volumes d'un gradient linéaire de 0 % à 60 % d'un tampon phosphate 20 mM pH=7,4, 500 mM NaCl, 50 mM EDTA. A la fin de l'élution la colonne est lavée par 6 volumes de tampon phosphate 20 mM pH=7,4, 1 M NHCl₄.

La colonne peut à ce stade être activée pour un nouveau passage. La **fig**. **3** représente le profil de chromatographie : la courbe a représente l'absorbance à 280nm des protéines, la courbe b représente la conductivité des solutions injectées, la courbe c le pourcentage de solution d'élution.

Afin de pouvoir utiliser la protéine en culture cellulaire, l'éluat est dans un premier temps concentré à l'aide de centricon plus-20 (Amicon, Millipor, France) possédant une membrane de séparation à 10 Kda, puis dialysé contre 30 volumes de PBS sans Mg²⁺ et Ca²⁺ à travers une membrane de dialyse possédant un point de coupure de 15 Kda. Après une nuit de dialyse à 4 °C, sous agitation douce, la protéine peut être concentrée à l'aide de centricon plus-20. La solution est ensuite conservée à - 20 °C.

Une fraction de la protéine est déposée en double sur un gel à 12% d'acrylamide SDS-page et mise à migrer comme précédemment décrit. Une partie du gel est transférée sur membrane de PVDF afin de révéler les protéines par chimiluminescence à l'aide d'un anticorps anti-histidine l'autre partie est colorée à l'argent par un kit Bio-rad silver stain afin d'obtenir le panel complet des protéines présentes dans l'échantillon.

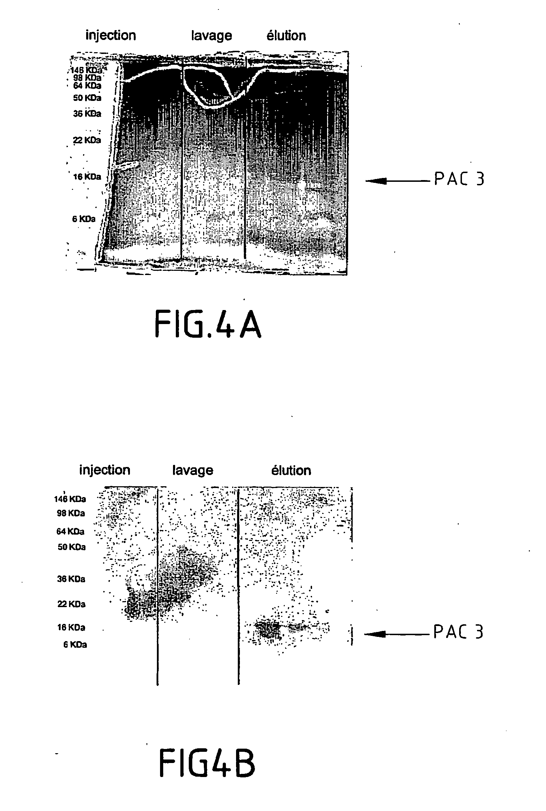

La protéine PAC3 ainsi isolée et purifiée possède en C-terminal une queue polyHistidine et est de séquence SEQ ID N° 3. On pourra se référer aux **fig. 4A** et **4B** qui montrent la protéine PAC3 de poids 16KDa.

### B - ACTIVITE BIOLOGIQUE DE LA PROTEINE PAC3

### B.1 - Inhibition de la prolifération de lignées tumorales pour lesquelles l'IL-6 exerce un effet mitogène

### B.1.1 - Choix des lignées et évaluation de leur prolifération.

Les cellules sont ensemencées à 2,5.10⁵ cellules.ml⁻¹ en milieu RPMT 1640 (Gibco) complémenté par 2 mM de L-glutamine, 100 UI.ml⁻¹ de pénicilline et 100 U.ml⁻¹ de streptomycine. 100 µl de cette suspension cellulaire sont "sevrés" 24 heures à 37 °C (5 % de CO₂ en atmosphère humique) en plaque 96 puits. Après "sevrage", le milieu de culture est remplacé par la solution à tester diluée dans du milieu de culture (RPMI 1640 complémenté par 2 mM de L-glutamine, 100 UI.ml⁻¹ de pénicilline, 100 U.ml⁻¹ de streptomycine et 20 % de sérum de veau nouveau né). L'ensemble est mis à incuber 24, 48 ou 72 h (37 °C à 5 % de CO₂ en atmosphère humique) suivant les conditions de manipulation. La prolifération cellulaire est déterminée par incorporation de thymidine tritiée.

U266 est une lignée de myélome ; il est clairement établi que l'IL-6 dans ce modèle joue un rôle autocrine dans la prolifération et que la cellule exprime de grandes quantités de récepteurs gp 80 et gp 130 à sa surface cellulaire (contrairement aux lignées d'adénocarcinome du rein qui n'expriment pas gp 80 à leur surface).

### B.1.2 - Résultats

### B.1.2.1 - Activité de la protéine PAC3 de SEQ ID N° 3 sur la prolifération de la lignée U266

Le contrôle négatif utilisé est une production de baculovirus codant pour une protéine non pertinente (une enzyme dégradant le catéchol ref pAcHLT-XylE "PAC-XylE"contrôle vector de Pharmigen) purifiée dans les mêmes conditions que la protéine PAC3 selon l'invention.

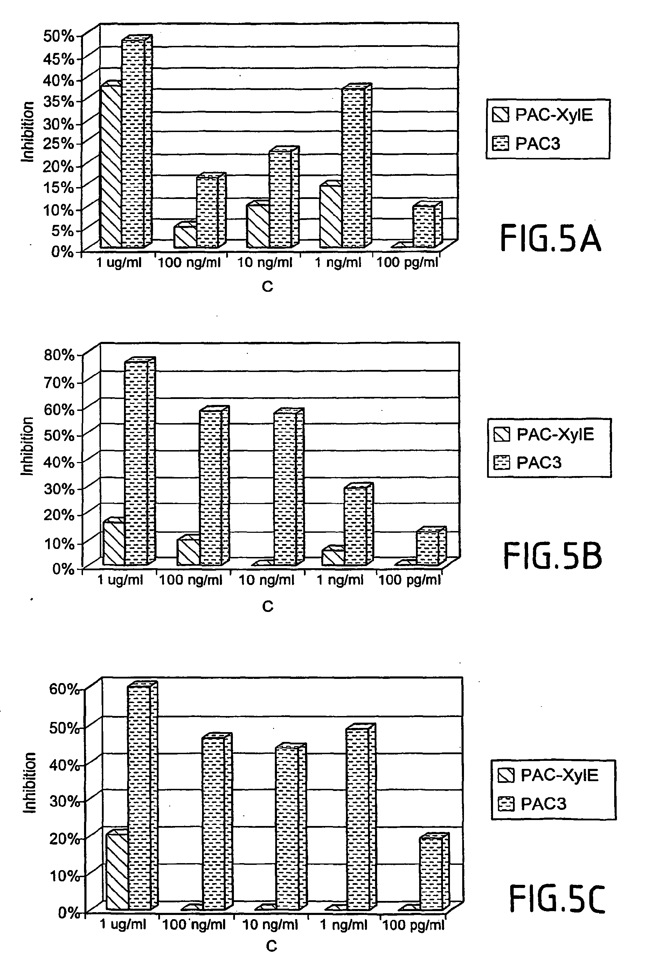

La **fig. 5A** montre l'évolution du pourcentage d'inhibition, en fonction de la concentration protéique (c), de la prolifération de U266 sans activation, en présence de la protéine PAC3.

**La** **fig. 5B** montre l'évolution du pourcentage d'inhibition, en fonction de la concentration protéique (c), de la prolifération de U266 avec activation par 1,26 pg/ml d'IL-6 en présence de la protéine PAC-3.

La **fig. 5C** montre l'évolution du pourcentage d'inhibition, en fonction de la concentration protéique (c), de la prolifération de U266 avec activation par 12,6 pg/ml d'IL-6 en présence de la protéine PAC-3.

Comme le montrent les graphiques présentés à la **fig. 5A, 5B et 5C**, avec la protéine PAC3, une inhibition de la prolifération jusqu'à 25% par rapport au contrôle est obtenue. Le caractère limité de cette inhibition résulte probablement d'un mode de fonctionnement majoritairement intracrine de l'IL-6 dans cette lignée, n'impliquant pas les récepteurs de l'IL-6 exprimés à la surface cellulaire.

Cependant la magnitude de cette inhibition induite spécifiquement par la protéine PAC3 selon l'invention est majorée lorsque les cellules sont cultivées en présence d'IL-6 exogène, atteignant 60% du niveau de prolifération observé en présence d'IL-6 exogène. Ceci confirme la capacité de la protéine PAC3 selon l'invention à bloquer significativement l'activité biologique de l'IL-6 notamment l'induction de la prolifération tumorale induite par cette IL-6 exogène.

Au total, ces résultats indiquent que la protéine PAC3 de **SEQ ID N° 3** selon l'invention bloque la prolifération induite par l'IL-6 exogène de la lignée tumorale U266, en l'absence d'IL-6, mais surtout inhibe de manière majeure la prolifération de cette lignée induite par l'IL-6 exogène. Ces résultats confirment l'effet antagoniste de l'IL-6 de la protéine PAC3 de **SEQ ID N° 3** selon l'invention.

### B.2 - Différenciation des progéniteurs CD34+

### B.2.1 - Méthodes

Des prélèvements de sang de cordon ombilical sont dilués au 1/2 en PBS, puis déposés sur une solution de ficoll de densité 1,077 afin de séparer par centrifugation en fonction de la densité les différents constituants du prélèvement. Les cellules mononucléées se situant à l'interface, celles-ci sont ponctionnées du ficoll afin de purifier les CD34+ par sélection positive. La purification débute par un marquage à l'aide d'un anticorps monoclonal de souris anti-CD34 (anticorps monoclonal IgG1 de souris Coulter, Marseille, France), puis par un second anticorps monoclonal de chèvre anti-F(ab')2 de souris (Macs, Tebu, Paris, France) couplé à une bille magnétique. Le complexe formé est purifié sur une colonne magnétique.

Après purification, les progéniteurs CD34+ sont cultivés de J0 à J6 en présence de RPMI 1640 classique complémenté de TNFα (2,5 ng/ml), GM-CSF (100ng/ml), SCF (25ng/ml) et du sérum AB+ humain (2%) (EFS, Lyon, France). A J6, les CD34+ sont lavés 3 fois en PBS pour éliminer le sérum AB+, puis cultivés en présence de TNFα (2,5 ng/ml), GM-CSF (100 ng/ml) et différentes concentrations de solution à tester.

A J12, les cellules sont numérées et lavées, puis mises à incuber en présence d'anticorps fluorescent 20 min. à 4 °C. Après l'incubation les cellules sont lavées 3 fois en PBS puis analysées par cytométrie en flux.

### B.2.2 - Résultats

La protéine PAC3 de **SEQ ID N° 3** selon l'invention exerce deux types d'activités sur la différenciation des progéniteurs CD34+ en cellules dendritiques et en présence de GM-CSF et TNF α :
- la première est une action inhibitrice de l'effet de l'IL-6 sur l'expression de marqueurs membranaires de différenciation des DC notamment du CD 14,
- la seconde correspond à un blocage partiel par la protéine PAC3 de **SEQ ID N°2** de l'acquisition de CD1a par les cellules en cours de différenciation.

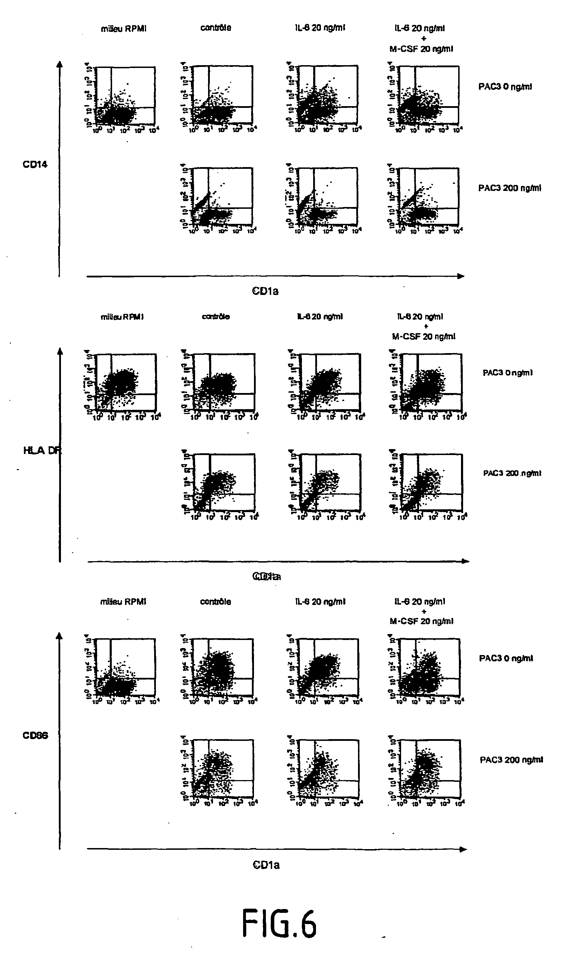

En effet, comme le montrent les résultats présentés à la **fig. 6** le maintien de l'expression du CD14 par l'IL-6 ou IL-6 combiné avec le M-CSF est inhibée en présence de la protéine PAC3 de **SEQ ID N° 3**. Il en est de même pour l'inhibition de l'expression de CD86 induite par IL-6 ou par la combinaison IL-6 et MCSF, qui est partiellement restaurée en présence de la protéine PAC3 de **SEQ ID N° 3.** Ces résultats confirment l'action inhibitrice de la protéine PAC3 de **SEQ ID N° 3** sur l'activité biologique de l'IL-6, dans un modèle pertinent en pathologie clinique chez l'homme, qui est le blocage par l'IL-6 de la différenciation des DC à partir de progéniteurs CD34+ .

La protéine PAC3 de **SEQ ID N° 3** affecte donc la différenciation des cellules dendritiques, orientant leur engagement vers une sous-population exprimant de manière plus faible le marqueur CD1a. La protéine PAC3 **de SEQ ID N° 3** bloque en outre les effets immunosuppresseurs de l'IL-6 dans ce modèle, notamment sa capacité à inhiber la différenciation normale des cellules dendritiques.

### B.3 - Conclusions

La protéine PAC3 de **SEQ ID N° 3** est ainsi capable de bloquer spécifiquement la prolifération induite par l'IL-6 de lignées cellulaires tumorales, de la lignée de myélome U266. La protéine PAC3 de **SEQ ID N° 3** bloque également la capacité de l'IL-6 à inhiber la différenciation des cellules dendritiques *in vitro.*

### C - PRODUCTION ET PURIFICATION DE LA PROTEINE DE SEQUENCE SEQ ID N° 4

La position de la queue polyhistidine utilisée pour la purification a été modifiée. Ainsi celle-ci est placée en *N-*terminal de la protéine, afin d'être clivée par le système TAGZyme après purification.

### C.1- Préparation du vecteur de transfert

Les mutations sont obtenues à l'aide du plasmide KAE15.8 précédemment obtenu et séquencé, sur lequel est réalisée une PCR à l'aide du couple d'amorces de séquences :
- **SEQ ID N° 17** La séquence **SEQ ID N° 17** crée en 5' un site de restriction BanHI et une queue poly-histidine compatible avec le système TACZyme de séquence peptidique MKHHHHHHQ.
- **SEQ ID N° 18**
   GGT GCG AAT TCTrACA TTT GCC GAA GAG CCC TC
   La séquence **SEQ ID N° 18** crée en 3' un site de restriction EcoRI après le codon stop.
   La PCR, l'analyse des fragments et leur purification sont réalisées comme précédemment décrit aux points A .1. et A.2.
   La digestion des fragments PCR purifiés et du plasmide de transfert pAcGP67-B est réalisée par les enzymes BamHI et EcoRI (biolab) pendant 4 heures à 37°C. L'ensemble des autres étapes de construction du baculovirus sont semblables aux étapes décrites aux points A.3.2 à A.3.3 ci-dessus.

### C.2 - Production et purification de la protéine PAC3Bis

### C.2.1 - Production

Celle-ci est réalisée par infection de cellules High Five (Invitrogen, France) en suspension. 1 1 de milieu de culture EXPRESS FIVE SFM (Invitrogen, France) est ensemencé à l'aide de 2.10⁶ cellules.ml⁻¹ et 1 ml de suspension virale à 10⁸ pfu.ml⁻¹. L'ensemble est mis à incuber pendant 7 jours à 27°C, sous agitation douce. A la fin de la culture, les cellules sont culottées par une centrifugation à 10 000 g pendant 5 minutes et le surnageant est stocké à 4°C.

### C.2.2.1- Chromatographie

La colonne est montée sur le système de chromatographie HR de Biorad stabilisé à 4° C en chambre froide. Le débit de la colonne est limité à 3 ml.min⁻¹ avec une pression maximum de 150 psi.

Dans un premier temps, la colonne est lavée et activée par un premier lavage par 5 volumes d'eau distillée, puis activée par 0,5 volume d'une solution de sulfate de cuivre à 100 mM. Le cuivre non fixé est éliminé par 10 volumes d'eau distillée. La colonne est stabilisée par 10 volumes de tampon phosphate 20 mM pH = 7,4, 1M NH₄Cl.

La purification se fait à l'aide de plusieurs passages successifs afin de ne pas saturer la colonne.

Chaque passage débute par une stabilisation de la ligne de base des U.V. à 280 nm à l'aide de 2 volumes de tampon phosphate 20 mM pH = 7,4, 1M NH₄Cl, puis 4 volumes de dialysat sont injectés. Un lavage est réalisé par 6 volumes de tampon phosphate 20 mM pH = 7,4, 1M NH₄Cl.

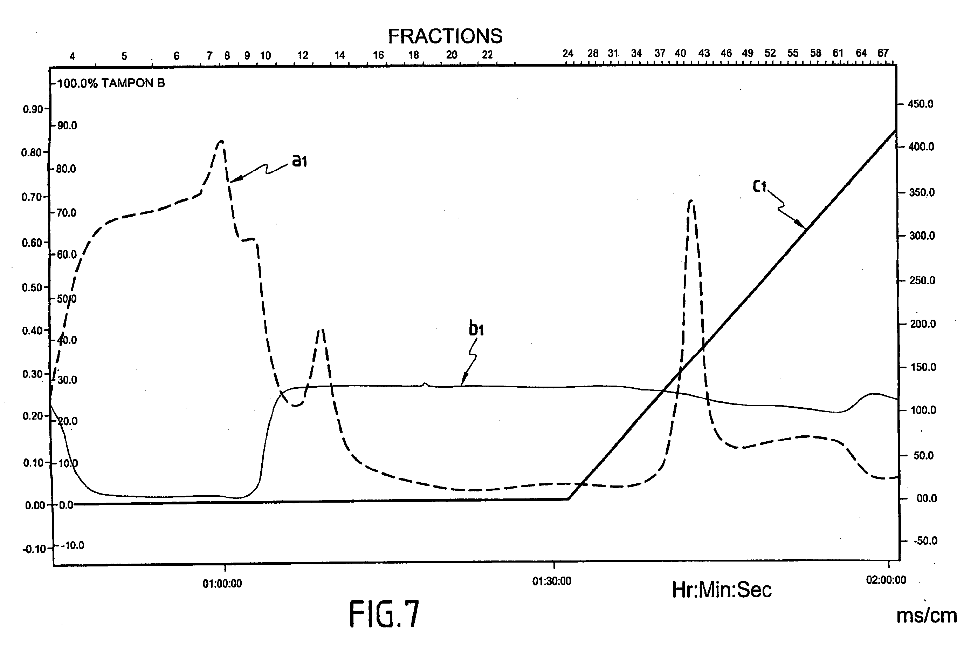

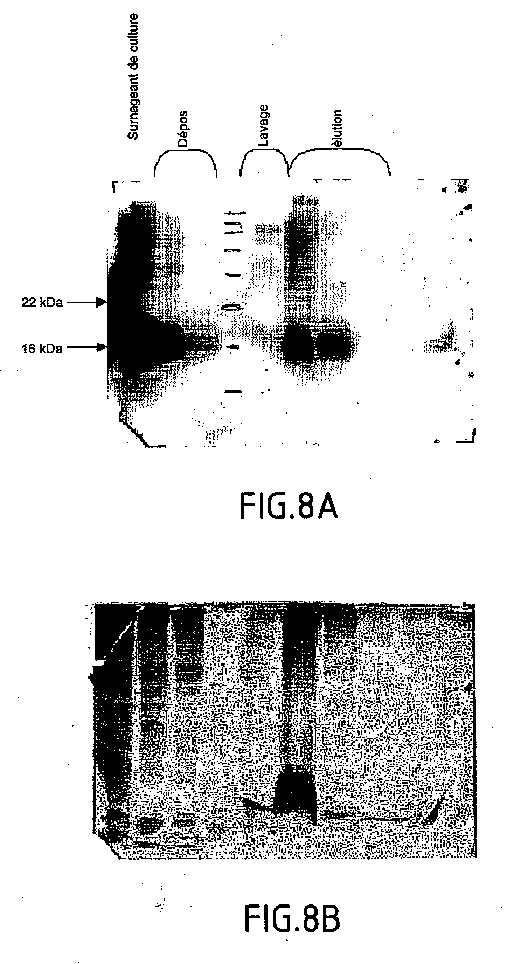

Les protéines sont éluées de la colonne à l'aide de 4 volumes d'un gradient linéaire de 0 % à 60 % d'un tampon phosphate 20 mM pH = 7,4, 500 mM NaCl, 500 mM d'imidazole. A la fin de l'élution, la colonne est inactivée par injection de 6 volumes de tampon phosphate 20 mM pH = 7,4, 500 mM EDTA puis lavée par 6 volumes d'eau. A ce stade, la colonne peut à nouveau être activée pour une nouvelle chromatographie. Le profil de chromatographie est présenté à la **Fig. 7** : la courbe **a**₁ représente l'absorbance à 280 nm, la courbe **b**₁ représente la conductivité des solutions et la courbe **c₁** le pourcentage de solution d'élution. L'analyse des fractions de la chromatographie est réalisée par Western blot comme décrit au point A.4. précédent. Différentes fractions sont déposées sur deux gels SDS page à 15 % d'acrylamide. Après migration, un premier est transféré sur une membrane PVDF pour être révélé à l'aide d'anticorps anti-histidine et chimiluminescence (**Fig. 8A**), tandis que le second est coloré à l'argent (**Fig. 8B**).

### C.2.3 - Digestion de la queue poly-histidine par le système TAGZyme

Une fois les fractions d'intérêt identifiées, celles-ci sont dialysées contre 30 volumes de tampon de digestion TAGyme (20 mM NaH₂PO₄, 1,5 M NaCl pH 7) à travers une membrane de dialyse possédant un point de coupure de 8 Kda. Après une nuit de dialyse à 4°C, sous agitation douce. La protéine peut alors être concentrée à l'aide de centricon plus-20 puis dosée par dosage DC de biorad (Marne la Coquette, France).

### Principe du système TAGZyme :

La queue poly-histidine est clivée de la protéine par une succession de trois étapes enzymatiques. Dans un premier temps, une di-peptidase (DAPase) clive des di-peptides en *N-*terminal de la protéine pour s'arrêter au niveau d'une glutamine. Dans un second temps, cette glutamine est transformée en pyroglutamate par une Qcyclase, afin que celui-ci soit clivé par une troisième enzyme la pGAPase pour obtenir la protéine native. L'ensemble de ces trois enzymes possède une queue histidine en *C*-terminal permettant ainsi de les extraire de la préparation protéique après digestion.

5 mg de protéine à cliver sont incubés 15 minutes à 37° C en présence de 250 mU de DAPaze activée par 10 mM de cystéamine-HCl et 15 U de Qcyclase. Le produit de digestion est appliqué sur une colonne de 1ml de NI-NTA agarose préalablement lavée par 5 volumes de tampon de digestion TAGZyme. Une fois le produit de digestion complètement passé à travers la colonne, celle-ci est lavée une seconde fois par deux volumes de tampon TAGZyme. L'ensemble des fractions passant à travers la colonne est récolté pour être clivé par une seconde incubation de 90 minutes à 37°C en présence de 1 mU de pGAPase activée par 2 mM de cystéamine-HCl. La digestion terminée, la préparation protéique est une seconde fois purifiée par une colonne de 1 ml de NI-NTA comme précédemment décrit.

Afin de rendre compatible la préparation protéique au test biologique, celle-ci est dialysée contre 20 volumes de PBS sans Ca²⁺ et Mg²⁺ à travers une membrane de dialyse de 8 kDa de point de coupure.

Le résultat de digestion est analysé à l'aide de gel d'acrylamide coloré à l'argent et par Western blot comme précédemment décrit au A.3.3.

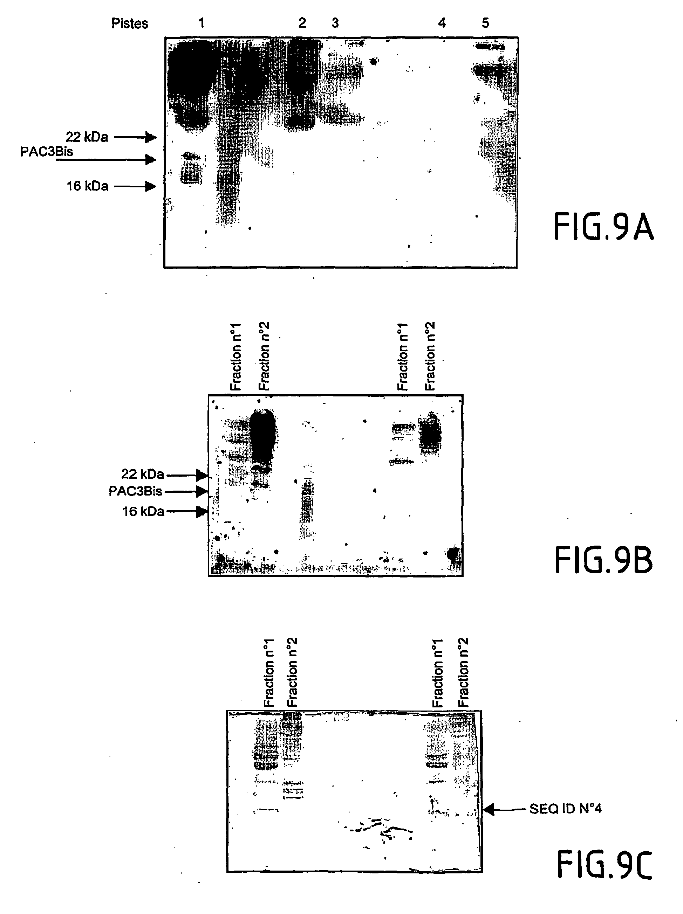

La **Fig. 9** **A** représente une autoradiographie du western blot révélée par un anticorps anti-histidine des différentes étapes de digestion. Dans l'ordre des dépôts, sont présentés en **1)** la protéine non digérée, en **2) 3)** les retentats respectivement de la 1° et de la 2° colonne de purification, en **4)** et **5)** les éluats respectivement de la 1° et de la 2° colonne de purification. Les retentats des colonnes de purification démontrent bien que celles-ci capturent une grande partie des protéines possédant une queue poly-histidine **(****Fig. 9** **A :** pistes 3 et 4) et les protéines partiellement digérées (**Fig. 9** **A** : piste 3). Les pistes 4 et 5 ne présentent plus de protéine poly-histidine au poids moléculaire attendu de PAC3Bis.

Les **Fig. 9B** et **9C** représentent deux fractions de pureté différente de PAC3Bis avant et après digestion enzymatique par le système TAGZyme en Westen Blot et après révélation à l'argent respectivement. A gauche du gel révélé à l'argent ou de l'auto radiogramme western blot, sont déposées les protéines non clivées et à droite du gel révélé à l'argent ou de l'auto radiogramme sont déposées les protéines après digestion.

Sur ces gels, il est possible de constater que bien que plus de marquage poly-histidine ne soit observé (photo de droite), on observe toujours la protéine sur un gel coloré à l'argent (photo de gauche). Ces résultats démontrent que la protéine PAC3Bis n'est pas dégradée, mais qu'elle ne possède plus de queue poly-histidine et a donc la séquence **SEQ ID N° 4.**

### D - ACTIVITE BIOLOGIQUE

### Inibition de la prolifération de lignées tumorales pour lesquelles l'IL-6 exerce un effet mitogène

Les tests de prolifération sont identiques à ceux mentionnés au point B.1.

Les cellules de myélome U266 sont incubées pendant 48 heures en présence de doses croissantes de protéine PAC3Bis (**SEQ ID N°19**) ou de protéine de séquence **SEQ ID N°4**. La prolifération cellulaire est déterminée par 16 heures d'incorporation de thymidine tritiée. Les résultats sont exprimés en pourcentage d'inhibition par rapport à une prolifération de U266 en absence de PAC3Bis ou de protéine de **SEQ ID N° 4**.

### Résultats

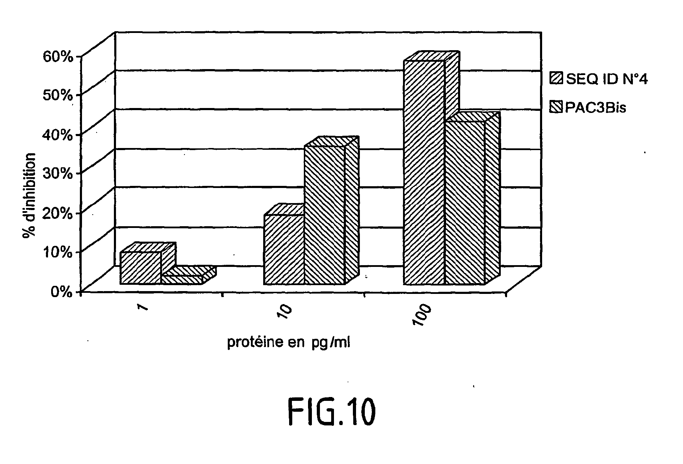

Le même taux d'inhibition est observé avec la protéine PAC3Bis qu'avec PAC3 sur la prolifération d'U266 en absence d'IL-6. L'absence de différence significative dans l'inhibition en présence (PAC3Bis) ou non de la queue poly-histidine (**SEQ ID N° 4**) démontre que la queue polyhistidine n'interfère pas dans l'activité biologique de la protéine (**Fig.10**).

## Revendications

1. Utilisation d'une protéine choisie parmi la protéine de séquence **SEQ ID N° 4**, la protéine PAC3 de séquence **SEQ ID N°3** et la protéine PAC3Bis de séquence **SEQ ID N°19**, pour la fabrication d'un médicament destiné à inhiber l'activité de l'IL-6.

2. Utilisation selon la revendication 1 pour la fabrication d'un médicament anti-inflammatoire.

3. Utilisation selon la revendications 1 pour la fabrication d'un médicament anticancéreux.

4. Utilisation selon la revendication 3, pour la fabrication d'un médicament destiné à traiter les lymphomes, leucémies, myélomes, carcinomes, sarcomes, le sarcome de Kaposi ou la maladie de Castslman.

5. Utilisation selon la revendication 4, pour la fabrication d'un médicament destiné à traiter les myélomes.

6. Utilisation selon la revendication 1 pour la fabrication d'un médicament destiné à inhiber la prolifération des cellules tumorales bénignes ou malignes dont la croissance est stimulée par l'IL-6 ; ou à induire la mort des cellules dont la survie est dépendante de la présence d'IL-6 ; ou encore à inhiber la dissémination métastatiqué des cellules tumorales dont la dissémination est induite par l'IL-6, de manière autocrine, paracrine ou endocrine ; ou à inhiber l'effet protecteur de l'IL-6 sur la mort des cellules tumorales induite par les agents cytotoxiques (chimiques, biologiques ou physiques) ou cytostatiques ; ou à inhiber les propriétés proinflammatoires, cachectisantes et régulatrices de l'hématopoïèse de l'IL-6 résultant d'une production anormale de cette protéine ; ou à inhiber les remaniements osseux, notamment l'ostéolyse, induits par l'IL-6, dans les maladies dégénératives, inflammatoire ou tumorales, par exemple ; ou à inhiber les propriétés immunologiques de l'IL-6 immunostimulantes ou immunosuppressives; ou à traiter les syndromes dysimmunitaires.

7. Utilisation selon la revendication 6 pour la fabrication d'un médicament destiné à traiter la polyarthrite ou le lupus érythémateux disséminé.

8. Composition pharmaceutique contenant une protéine choisie parmi la protéine de séquence **SEQ ID N° 4**, la protéine PAC3 de séquence **SEQ ID N°3** et la protéine PAC3Bis de séquence **SEQ ID N°19,** en association avec un excipient pharmaceutiquement acceptable convenable.

9. Composition pharmaceutique selon la revendication 8 **caractérisée en ce qu'**elle est destinée à une administration orale, sublinguale, sous-cutanée, intramusculaire, intra-veineuse ou topique.

10. Protéine PAC3 à activité inhibitrice de l'IL-6, **caractérisée en ce qu'**elle consiste en la **SEQ ID N° 3**.

11. Protéine à activité inhibitrice de l'IL-6, **caractérisée en ce qu'**elle consiste en la **SEQ ID N°4.**

12. Protéine PAC3Bis à activité inhibitrice de l'IL-6, **caractérisée en ce qu'**elle consiste en la **SEQ ID N° 19.**

13. Séquences d'acides nucléiques codant pour la protéine PAC3 selon la revendication 10, la protéine PAC3Bis selon la revendication 12, ou la protéine de **SEQ ID N° 4** selon la revendication 11.

14. Protéine PAC3 selon la revendication 10, la protéine PAC3Bis selon la revendication 12, ou protéine de **SEQ ID N° 4** selon la revendication 11 pour leur utilisation en tant que médicament.

15. Animaux transgéniques, à l'exclusion de l'homme, exprimant un transgène de la protéine PAC3 selon la revendication 10, de la protéine PAC3Bis selon la revendication 12, ou de la protéine de **SEQ ID N° 4** selon la revendication 11.

16. Vecteur d'expression, **caractérisé en ce qu'**il comprend une séquence d'acides nucléiques selon la revendication 13 et les moyens nécessaires à son expression.

17. Microorganismes ou cellules hôtes transformés par un vecteur d'expression selon la revendication 16.

## Claims

1. Use of a protein selected from the protein of sequence **SEQ ID N°4**, the PAC3 protein of sequence **SEQ ID N°3**, and the PAC3Bis protein of **SEQ ID N°19**, for the preparation of a drug intended to inhibit the activity of IL-6.

2. Use as in claim 1 for the preparation of an anti-inflammatory drug.

3. Use as in claim 1 for the preparation of an anti-cancer drug.

4. Use as in claim 3 for the preparation of a drug intended to treat lymphomas, leukemias, myelomas, carcinomas, sarcomas, Kaposi's sarcoma or Castelman's disease.

5. Use as in claim 4 for the preparation of a drug intended to treat myelomas.

6. Use as in claim 1 for the preparation of a drug intended to inhibit the proliferation of benign or malignant tumour cells whose growth is stimulated by IL-6 ; or to induce the death of cells whose survival is dependent on IL-6 presence ; or further to inhibit the metastatic dissemination of tumour cells whose dissemination is induced by IL-6, in autocrine, paracrine or endocrine manner ; to inhibit the protective effect of IL-6 on the death of tumour cells induced by cytotoxic (chemical, biological or physical) or cytostatic agents ; or to inhibit the proinflammatory, cachectising and regulating properties of IL-6 hematopoiesis resulting from abnormal production of this protein ; or to inhibit bone changes, IL-6-induced osteolysis in particular, in degenerative, inflammatory or tumoral diseases for example ; or to inhibit the immunostimulant or immunosuppressive immunological properties of IL-6 ; or to treat dysimmune syndromes.

7. Use as in claim 6 for the preparation of a drug intended to treat polyarthritis or systemic lupus erythematosus.

8. Pharmaceutical composition containing a protein selected from the protein of sequence **SEQ ID N°4**, the PAC3 protein of sequence **SEQ ID N°3**, and the PAC3Bis protein of **SEQ ID N°19**, in association with a pharmaceutically acceptable excipient.

9. Pharmaceutical composition as in claim 8, **characterized in that** it is entended to oral, sublingual, sub-cutaneous, intramuscular, intra-venous, or topical administration.

10. PAC3 protein with IL-6 inhibiting activity, **characterized in that** it consists of **SEQ ID N°3.**

11. Protein with IL-6 inhibiting activity, **characterized in that** it consists of **SEQ ID N°4.**

12. PAC3Bis protein with IL-6 inhibiting activity, **characterized in that** it consists of **SEQ ID N°19.**

13. **Nucleic** acid sequences encoding the PAC3 protein as defined in claim 10, the PAC3Bis protein as in claim 12, or the protein of **SEQ ID N°4** as in claim 11.

14. PAC3 protein as defined in claim 10, the PAC3Bis protein as defined in claim 12, or the protein of **SEQ ID N°4** as in claim 11, for their use as therapeutic drug.

15. Transgenic animals, with the exception of man, expressing a transgene of the PAC3 protein as defined in claim 10, of the PAC3Bis protein as defined in claim 12, or of the protein of **SEQ ID N°4** as defined in claim 11.

16. Expression vector, **characterized in that** it comprises a sequence of nucleic acids as defined in claim 13 and the necessary means for its expression.

17. Micro-organisms or host cells transformed by an expression vector as defined in claim 16.

## Patentansprüche

1. Verwendung eines Proteins, ausgewählt aus dem Protein mit der Sequenz SEQ ID Nr. 4, dem Protein PAC3 mit der Sequenz SEQ ID Nr.3 und dem Protein PAC3Bis mit der Sequenz SEQ ID Nr.19, für die Herstellung eines Medikaments, welches dazu bestimmt ist, die Aktivität von IL-6 zu hemmen.

2. Verwendung nach Anspruch 1, für die Herstellung eines entzündungshemmenden Medikaments.

3. Verwendung nach Anspruch 1, für die Herstellung eines krebshemmenden Medikaments

4. Verwendung nach Anspruch 3, für die Herstellung eines Medikaments, das für die Behandlung von Lymphomen, Leukämien, Myelomen, Karzinomen, Sarkomen, dem Kaposi-Sarkom oder der Castleman-Krankheit bestimmt ist.

5. Verwendung nach Anspruch 4, für die Herstellung eines Medikaments, das für die Behandlung von Myelomen bestimmt ist.

6. Verwendung nach Anspruch 1, für die Herstellung eines Medikaments, das dazu bestimmt ist, die Proliferation der benignen oder malignen Tumorzellen zu hemmen, deren Wachstum durch das IL-6 stimuliert wird; oder den Tod der Zellen zu induzieren, deren Überleben von der Präsenz des IL-6 abhängt; oder aber die metastatische Dissemination der Tumorzellen zu hemmen, deren Dissemination durch das IL-6 autokrin, parakrin oder endokrin induziert wird; oder die Schutzwirkung des IL-6 auf den Tod der Tumorzellen zu hemmen, der durch die zytotoxischen (chemischen, biologischen oder physikalischen) oder zytostatischen Wirkstoffe induziert wird; oder die proinflammatorischen, kachektisierenden und die Hämatopoese des IL-6 regulierenden Eigenschaften zu hemmen, die aus einer anormalen Produktion dieses Proteins resultieren; oder den durch IL-6 induzierten Knochenumbau, insbesondere Osteolyse, beispielsweise bei degenerativen, inflammatorischen oder tumoralen Krankheiten zu hemmen; oder die immunologischen Eigenschaften des IL-6, immunstimulierenden oder immunsuppressiven, zu hemmen; oder Immundysfunktionen zu behandeln.

7. Verwendung nach Anspruch 6, für die Herstellung eines Medikaments, das für die Behandlung von Polyarthritis oder disseminiertem Lupus erythematodes bestimmt ist.

8. Pharmazeutische Zusammensetzung, die ein Protein ausgewählt aus dem Protein mit der Sequenz SEQ ID Nr. 4, dem Protein PAC3 mit der Sequenz SEQ ID Nr. 3 und dem Protein PAC3Bis mit der Sequenz SEQ ID Nr. 19, in Verbindung mit einem geeigneten pharmazeutisch akzeptablen Trägerstoff enthält.

9. Pharmazeutische Zusammensetzung nach Anspruch 8, **dadurch gekennzeichnet, daß** sie für eine orale, sublinguale, subkutane, intramuskuläre, intravenöse oder topische Verabreichung bestimmt ist.

10. Protein PAC3 mit IL-6-hemmender Wirkung, **dadurch gekennzeichnet, daß** es aus der SEQ ID Nr. 3 besteht.

11. Protein mit IL-6-hemmender Wirkung, **dadurch gekennzeichnet, daß** es aus der SEQ ID Nr. 4 besteht

12. Protein PAC3Bis mit IL-6-hemmender Wirkung, **dadurch gekennzeichnet, daß** es aus der SEQ ID Nr. 19 besteht

13. Nukleinsäuresequenzen, die für das Protein PAC3 nach Anspruch 10, das Protein PAC3Bis nach Anspruch 12 oder das Protein mit der SEQ ID Nr. 4 nach Anspruch 11 kodieren.

14. Protein PAC3 nach Anspruch 10, das Protein PAC3Bis nach Anspruch 12 oder Protein der SEQ ID Nr. 4 nach Anspruch 11 für ihre Verwendung als Medikament.

15. Transgene Tiere, unter Ausschluß des Menschen, die ein Transgen des Proteins PAC3 nach Anspruch 10, des Proteins PAC3Bis nach Anspruch 12 oder des Proteins mit der SEQ ID Nr.4 nach Anspruch 11 exprimieren.

16. Expressionsvektor, **dadurch gekennzeichnet, daß** er eine Nukleinsäuresequenz nach Anspruch 13 sowie die für deren Expression notwendigen Mittel umfaßt.

17. Mikroorganismen oder Wirtszellen, die durch einen Expressionsvektor nach Anspruch 16 transformiert sind.


### SEQUENCE LISTING

<110> Université Claude Bernard Lyon 1
   Centre Léon Bérard
   INSERM
   Hospices Civils de Lyon
<120> Nouvelles protéines à activité inhibitrice de l'IL-6
<130> 1H708400-BWO-c22
<140> PCT/FR 02/04171
   <141> 2002-12-04
<150> FR 01/15 623
   <151> 2001-12-04
<160> 19
<170> PatentIn version 3.1
<210> 1
   <211> 212
   <212> PRT
   <213> Homo sapiens
<400> 1
   Met Asn Ser Phe Ser Thr Ser Ala Phe Gly Pro Val Ala Phe Ser Leu
   1 5 10 15
   Gly Leu Leu Leu Val Leu Pro Ala Ala Phe Pro Ala Pro Val Pro Pro
   20 25 30
   Gly Glu Asp Ser Lys Asp Val Ala Ala Pro His Arg Gln Pro Leu Thr
   35 40 45
   Ser Ser Glu Arg Ile Asp Lys Gln Ile Arg Tyr Ile Leu Asp Gly Ile
   50 55 60
   Ser Ala Leu Arg Lys Glu Thr Cys Asn Lys Ser Asn Met Cys Glu Ser
   65 70 75 80
   Ser Lys Glu Ala Leu Ala Glu Asn Asn Leu Asn Leu Pro Lys Met Ala
   85 90 95
   Glu Lys Asp Gly Cys Phe Gln Ser Gly Phe Asn Glu Glu Thr Cys Leu
   100 105 110
   Val Lys Ile Ile Thr Gly Leu Leu Glu Phe Glu Val Tyr Leu Glu Tyr
   115 120 125
   Leu Gln Asn Arg Phe Glu Ser Ser Glu Glu Gln Ala Arg Ala Val Gln
   130 135 140
   Met Ser Thr Lys Val Leu Ile Gln Phe Leu Gln Lys Lys Ala Lys Asn
   145 150 155 160
   Leu Asp Ala Ile Thr Thr Pro Asp Pro Thr Thr Asn Ala Ser Leu Leu
   165 170 175
   Thr Lys Leu Gln Ala Gln Asn Gln Trp Leu Gln Asp Met Thr Thr His
   180 185 190
   Leu Ile Leu Arg Ser Phe Lys Glu Phe Leu Gln Ser Ser Leu Arg Ala
   195 200 205
   Leu Arg Gln Met
   210
<210> 2
   <211> 1125
   <212> DNA
   <213> Homo sapiens
<400> 2
   ttctgccctc gagcccaccg ggaacgaaag agaagctcta tctcccctcc aggagcccag
   60
   ctatgaactc cttctccaca agcgccttcg gtccagttgc cttctccctg gggctgctcc
   120
   tggtgttgcc tgctgccttc cctgccccag tacccccagg agaagattcc aaagatgtag
   180
   ccgccccaca cagacagcca ctcacctctt cagaacgaat tgacaaacaa attcggtaca
   240
   tcctcgacgg catctcagcc ctgagaaagg agacatgtaa caagagtaac atgtgtgaaa
   300
   gcagcaaaga ggcactggca gaaaacaacc tgaaccttcc aaagatggct gaaaaagatg
   360
   gatgcttcca atctggattc aatgaggaga cttgcctggt gaaaatcatc actggtcttt
   420
   tggagtttga ggtataccta gagtacctcc agaacagatt tgagagtagt gaggaacaag
   480
   ccagagctgt gcagatgagt acaaaagtcc tgatccagtt cctgcagaaa aaggcaaaga
   540
   atctagatgc aataaccacc cctgacccaa ccacaaatgc cagcctgctg acgaagctgc
   600
   aggcacagaa ccagtggctg caggacatga caactcatct cattctgcgc agctttaagg
   660
   agttcctgca gtccagcctg agggctcttc ggcaaatgta gcatgggcac ctcagattgt
   720
   tgttgttaat gggcattcct tcttctggtc agaaacctgt ccactgggca cagaacttat
   780
   gttgttctct atggagaact aaaagtatga gcgttaggac actattttaa ttatttttaa
   840
   tttattaata tttaaatatg tgaagctgag ttaatttatg taagtcatat ttatattttt
   900
   aagaagtacc acttgaaaca ttttatgtat tagttttgaa ataataatgg aaagtggcta
   960
   tgcagtttga atatcctttg tttcagagcc agatcatttc ttggaaagtg taggcttacc
   1020
   tcaaataaat ggctaactta tacatatttt taaagaaata tttatattgt atttatataa
   1080
   tgtataaatg gtttttatac caataaatgg cattttaaaa aattc
   1125
<210> 3
   <211> 125
   <212> PRT
   <213> Artificial Sequence
<220>
   <223> traduction à partir du 2ème ATG du 3ème exon du gène de 1' IL-6, a vec queue polyhistidine
<400> 3
   Met Ala Glu Lys Asp Gly Cys Phe Gln Ser Gly Phe Asn Glu Glu Thr
   1 5 10 15
   Cys Leu Val Lys Ile Ile Thr Gly Leu Leu Glu Phe Glu Val Tyr Leu
   20 25 30
   Glu Tyr Leu Gln Asn Arg Phe Glu Ser Ser Glu Glu Gln Ala Arg Ala
   35 40 45
   Val Gln Met Ser Thr Lys Val Leu Ile Gln Phe Leu Gln Lys Lys Ala
   50 55 60
   Lys Asn Leu Asp Ala Ile Thr Thr Pro Asp Pro Thr Thr Asn Ala Ser
   65 70 75 80
   Leu Leu Thr Lys Leu Gln Ala Gln Asn Gln Trp Leu Gln Asp Met Thr
   85 90 95
   Thr His Leu Ile Leu Arg Ser Phe Lys Glu Phe Leu Gln Ser Ser Leu
   100 105 110
   Arg Ala Leu Arg Gln Met Tyr His His His His His His
   115 120 125
<210> 4
   <211> 118
   <212> PRT
   <213> Artificial Sequence
<220>
   <223> traduction à partir du 2ème ATG du 3ème exon du gène de 1' IL-6
<400> 4
   Met Ala Glu Lys Asp Gly Cys Phe Gln Ser Gly Phe Asn Glu Glu Thr
   1               5                   10                  15
   Cys Leu Val Lys Ile Ile Thr Gly Leu Leu Glu Phe Glu Val Tyr Leu
               20                  25                  30
   Glu Tyr Leu Gln Asn Arg Phe Glu Ser Ser Glu Glu Gln Ala Arg Ala
           35                  40                  45
   Val Gln Met Ser Thr Lys Val Leu Ile Gln Phe Leu Gln Lys Lys Ala
       50                  55                  60
   Lys Asn Leu Asp Ala Ile Thr Thr Pro Asp Pro Thr Thr Asn Ala Ser
   65                  70                  75                  80
   Leu Leu Thr Lys Leu Gln Ala Gln Asn Gln Trp Leu Gln Asp Met Thr
                   85                  90                  95
   Thr His Leu Ile Leu Arg Ser Phe Lys Glu Phe Leu Gln Ser Ser Leu
               100                 105                 110
   Arg Ala Leu Arg Gln Met
           115
<210> 5
   <211> 33
   <212> DNA
   <213> Artificial Sequence
<220>
   <223> amorce 5' crée un site de restriction HinD III en 5' du pr emier A TG du gène de l'IL-6
<400> 5
   gagaagcttt agcggccgcc caggagccca gct
   33
<210> 6
   <211> 34
   <212> DNA <213> Artificial Sequence
<220>
   <223> amorce 3' crée un site de restriction Sma I en 3' du codon stop d u gène de l'IL-6
<400> 6
   gaatgcccgg gaaactcgag aatctgaggt gccc
   34
<210> 7
   <211> 27
   <212> DNA
   <213> Artificial Sequence
<220>
   <223> amorce 5' qui crée un site de restriction BamHI en 5' du deuxième ATG du troisième exon du gène de l'IL-6
<400> 7
   ccttggatcc atggctgaaa aagatgg
   27
<210> 8
   <211> 48
   <212> DNA
   <213> Artificial Séquence
<220>
   <223> amorce 3' qui rend silencieux le codon stop de l'IL-6 puis crée u ne queue de 6 histidines avec un codon stop et un site de restric tion EcoR I sur le codon stop
<400> 8
   ggggaattct agtgatggtg atggtgatgg tacatttgcc gaagcccc
   48
<210> 9
   <211> 19
   <212> DNA
   <213> Artificial Sequence
<220>
   <223> amorce de séquence à partir de la base 30 dans le sens de lecture du gène de l'IL-6
<400> 9
   agctatgaac tccttctcc
   19
<210> 10
   <211> 20
   <212> DNA
   <213> Artificial Sequence
<220>
   <223> amorce de séquence à partir de la base 187 sur le brin complément aire du gène de l'IL-6 épissé
<400> 10
   gttctgaaga ggtgagtggc
   20
<210> 11
   <211> 21
   <212> DNA
   <213> Artificial Sequence
<220>
   <223> amorce de séquence à partir de la base 401 dans le sens de lectur e du gène de l'IL-6
<400> 11
   aggtatacct agagtacctc c
   21
<210> 12
   <211> 20
   <212> DNA
   <213> Artificial Sequence
<220>
   <223> amorce de séquence à partir de la base 697 sur le brin complément aire du gène de l'IL-6
<400> 12
   aactcgagaa tctgaggtgc
   20
<210> 13
   <211> 17
   <212> DNA
   <213> Artificial Sequence
<220>
   <223> amorce de séquence s'hybride en 5' du polylinker du plasmi de KS+
<400> 13
   tcgaggtcga cggtatc
   17
<210> 14
   <211> 20
   <212> DNA
   <213> Artificial Sequence
<220>
   <223> amorce de séquence s'hybride en 3' du polylinker du plasmi de KS+
<400> 14
   gcgagatctt gatcacctag
   20
<210> 15
   <211> 22
   <212> DNA
   <213> Artificial Sequence
<220>
   <223> amorce de séquence s'hybride en 5' du polylinker du plasmi de KS+
<400> 15
   gtaatacgac tcactatagg gc
   22
<210> 16
   <211> 20
   <212> DNA
   <213> Artificial Sequence
<220>
   <223> amorce de séquence à partir de la base 258 sur le brin complément aire du gène de l'IL-6
<400> 16
   gcttgttcct cactactctc
   20
<210> 17
   <211> 69
   <212> DNA
   <213> Artificial Sequence
<220>
   <223> crée en 5' un site de restriction BanHI et une queue poly-histidi ne compatible avec le système TAGZyme de séquence peptidiq ue MKHH HHHHQ
<400> 17
   gaggggatcc atgaaacacc atcaccatca ccatgctatg gctgaaaaag atggatgctt
   60
   ccaatctgg
   69
<210> 18
   <211> 32
   <212> DNA
   <213> Artificial Sequence
<220>
   <223> crée en 3' un site de restriction EcoRI après le codon sto P
<400> 18
   ggtgcgaatt ctacatttgc cgaagagccc tc
   32
<210> 19
   <211> 127
   <212> PRT
   <213> Artificial Sequence
<220>
   <223> traduction à partir du 2ème ATG du 3ème exon du gène de l'IL-6, av ec queue polyhistidine en N-terminal
<400> 19